# EUROPÄISCHE PATENTSCHRIFT

(11) **EP 0 782 991 B1**
(45) Veröffentlichungstag und Bekanntmachung des Hinweises auf die Patenterteilung: **17.04.2002**
(21) Anmeldenummer: 97100076.5
(22) Anmeldetag: 03.01.1997
(51) Int. Cl.: C07D 233/68

(54) **Verfahren zur Herstellung von 2-substituierten 5-Chlorimidazol-4-carbaldehyden**
Process for the preparation of 2-substituted 5-chloroimidazole-4-carbaldehydes
Procédé pour la préparation de 5-chloroimidazole-4-carbaldéhydes substitués sur la position 2

(30) Priorität: 05.01.1996 CH 2596
(43) Veröffentlichungstag der Anmeldung: 09.07.1997
(73) Patentinhaber: Lonza AG, 3930 Visp (CH)
(72) Erfinder: Griffiths, Gareth John, Dr., 3930 Visp/Wallis (CH); Stucky, Gerhard Christian, Dr., 3902 Brig-Glis/Wallis (CH)
(74) Vertreter: Weinhold, Peter, Dr.rer.nat.Dipl.Chem.

(56) Entgegenhaltungen:
- EP-A- 0 579 212
- EP-A- 0 614 891
- EP-A- 0 614 892
- EP-A- 0 653 422
- BULLETIN DE LA SOCIETE CHIMIQUE DE FRANCE, Nr. 3, 1971, Seiten 1040-51, XP002030297 R. JACQUIER ET AL.: "Recherches dans la série des azoles. LXXXI. - Etude de la tautomérie des imidazolinones-5(4)"

## Beschreibung

Die Erfindung beinhaltet ein neues Verfahren zur Herstellung von 5-Chlorimidazol-4-carbaldehyden der allgemeinen Formel 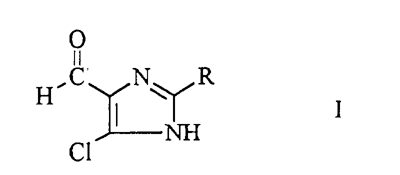 worin R Wasserstoff, eine Alkylgruppe, eine Alkenylgruppe, eine Cycloalkylgruppe oder eine gegebenenfalls substituierte Aryl- oder Arylalkylgruppe bedeutet.

5-Chlorimidazol-4-carbaldehyde der allgemeinen Formel I sind wichtige Zwischenprodukte zur Herstellung von blutdrucksenkenden Pharmazeutika (US-A-4 355 040) oder von herbizidwirksamen Verbindungen (DE-A-2 804 435).

Die EP-A-0 579 212 beschreibt ein Verfahren zur Herstellung von 5-Chlorimidazol-4-carbaldehyden, bei dem zunächst ein Glycinester mit einem Imidsäureester zu einem Dihydroimidazolin-4-on umgesetzt wird, das anschließend mit Phosphoroxychlorid in Gegenwart von N,N-Dimethylformamid in den gewünschten Carbaldehyd überführt wird.

R. Jacquier et al. (Bull. Soc. Chim. France, 1971 (3), 1040-51) beschreiben verschiedene Verfahren zur Herstellung von Imidazolinonen, bei denen u. a. von Glycinestern ausgegangen wird.

Bekannt ist ferner gemäss EP-A-0 614 892, ein Glycinesterhydrohalogenid mit einem Imidsäurester zu einem 3,5-Dihydroimidazol ringzuschliessen und anschliessend mit Phosphoroxychlorid in Gegenwart von N,N-Dimethylformamid in das Zielprodukt umzusetzen. Dieses Verfahren beinhaltet den Nachteil, dass der Glycinmethylester jeweils in situ aus dem entsprechenden Halogenid freigesetzt werden muss. Desweiteren ist das Glycinesterhydrohalogenid ein relativ teures Ausgangsprodukt.

Es bestand folglich die Aufgabe, ein wirtschaftliches Verfahren zu entwickeln, welches diesen Nachteilen Rechnung trägt und welches in der Lage ist, die Anforderungen an einen industriellen Prozess im technischen Masstab zu erfüllen.

Die Aufgabe konnte mit dem Verfahren gemäss Anspruch 1 gelöst werden.

Die allgemeinen Substituenten R, R₁, R₂ und R₃ haben folgende Bedeutung.

Alkylgruppe bedeutet eine geradkettige oder verzweigte (C₁-C₆)-Alkylgruppe, insbesondere Methyl, Ethyl, n-Propyl, i-Propyl, n-Butyl, i-Butyl, t-Butyl, Pentyl und seine Isomeren oder Hexyl und seine Isomeren. Bevorzugte Alkylgruppe für R ist die n-Butylgruppe. Bevorzugte Alkylgruppe für R₁ ist eine (C₁-C₄)-Alkylgruppe, besonders bevorzugt Methyl.

Alkenylgruppe bedeutet eine geradkettige oder verzweigte Alkenylgruppe mit bis zu sechs C-Atomen, insbesondere 1-Propenyl, 2-Propenyl, 1-Butenyl, 2-Butenyl, 3-Butenyl, Pentenyl und seine Isomeren oder Hexenyl und seine Isomeren. Bevorzugte Alkenylgruppe ist 2-Butenyl oder 3-Butenyl.

Cycloalkyl bedeutet Cyclopropyl, Cyclobutyl, Cyclopentyl oder Cyclohexyl.

Arylalkyl bedeutet Phenyl-(C₁-C₆)-alkyl, bevorzugt Benzyl. Aryl hat entsprechend die Bedeutung von Phenyl. Die Arylgruppe kann an ihrem aromatischen Kern einen oder mehrere Substituenten, nämlich (C₁-C₆)-Alkyl, Halogen, Nitro oder Amino aufweisen. Die Bezeichnung Halogen umfasst zweckmässig Chlor, Brom oder Iod, vorzugsweise Chlor.

Im ersten Schritt des erfindungsgemässen Verfahrens wird Glycin mit einem Imidsäureester der allgemeinen Formel 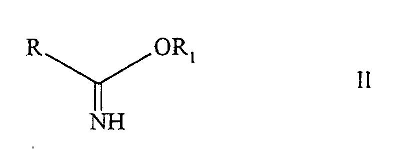 worin R die genannte Bedeutung hat und R₁ eine Alkylgruppe bedeutet, zu einer Verbindung der allgemeinen Formel 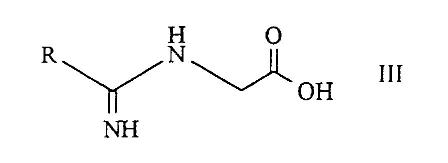 worin R die genannte Bedeutung hat, umgesetzt.

Zweckmässig erfolgt die Umsetzung in der ersten Stufe bei einem pH-Wert zwischen 4 bis 12, vorzugsweise bei 5 bis 9 und bei einer Temperatur in der Regel zwischen -20 °C und 80 °C, bevorzugt zwischen 0 °C und 30 °C.

Das Glycin liegt üblicherweise in einem geeigneten Lösungsmittel wie z.B. einem aliphatischen Alkohol wie Methanol oder Ethanol, gegebenenfalls gemischt mit Wasser, suspendiert vor. Der Imidsäureester kann in Form einer Lösung in einem inerten Lösungsmittel, wie z.B. in Toluol, Chlorbenzol oder einem aliphatischen Alkohol, zugegeben werden.

Die Reaktionsteilnehmer in der ersten Stufe werden zweckmässig stöchiometrisch eingesetzt. Nach einer Umsetzungszeit von zweckmässig 2 Stunden bis 48 Stunden kann die resultierende Verbindung der allgemeinen Formel III auf fachmännische Art und Weise aus dem Reaktionsgemisch isoliert werden, vorzugsweise erfolgt jedoch keine Isolierung, sondern direkte Weiterumsetzung zum Endprodukt.

In der zweiten und letzten Stufe wird die Verbindung der allgemeinen Formel III mit einem sogenannten Vilsmeier-Reagenz in das Endprodukt überführt.

Das Vilsmeier-Reagenz ist zweckmässig zusammengesetzt aus einem Chlorierungsmittel und einem Formamid der allgemeinen Formel 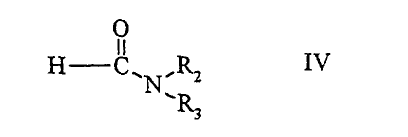 worin R₂ und R₃ gleich oder verschieden sind und eine (C₁-C₄)-Alkylgruppe oder eine Arylgruppe bedeuten. Bevorzugtes Formamid ist das N,N-Dimethylformamid.

Als Chlorierungsmittel werden zweckmässig Phosphoroxychlorid, Thionylchlorid, Phosgen oder Phosgen freisetzende Verbindungen, Phosphortrichlorid oder Phosphorpentachlorid verwendet. Bevorzugtes Chlorierungsmittel ist Phosphoroxychlorid.

Zweckmässig liegt das Molverhältnis Chlorierungsmittel zu Formamid der allgemeinen Formel IV im Vilsmeier-Reagenz zwischen 1 zu 1 und 4 zu 1.

Das Vilsmeier-Reagenz wird zweckmässig im Überschuss eingesetzt und dient dabei gleichzeitig als Lösungsmittel. Allerdings ist es auch möglich, ein inertes Lösungsmittel wie z.B. Toluol, Chlorbenzol oder Xylol zuzusetzen.

Die Umsetzungstemperatur für die Umsetzung in der zweiten Stufe liegt zweckmässig zwischen 60 °C und 200 °C.

Im Verlaufe dieser Umsetzung wird intermediär ein N,N-substituiertes Aminomethylenimidazolin der allgemeinen Formel 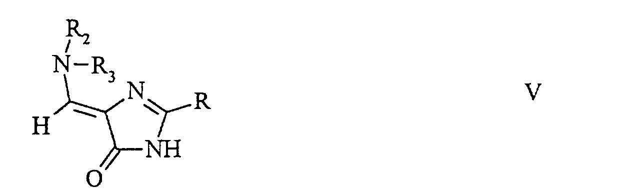 worin R, R₂ und R₃ die genannte Bedeutung haben, gebildet.

Diese Zwischenstufe ist Gegenstand der veröffentlichten europäischen Anmeldung EP-A-0 653 422.

Nach einer Reaktionszeit von in der Regel 1 Stunde bis 24 Stunden kann der entsprechende 5-Chlorimidazol-4-carbaldehyd auf fachmännische Weise, zweckmässig durch Behandeln des Reaktionsgemisches mit Wasser und Extraktion mit einem geeigneten Lösungsmittel, in guter Ausbeute und Reinheit gewonnen werden.

### Beispiel 1

### Herstellung von (Pentanimidoylamino)essigsäure

Eine gerührte Suspension von Glycin (18,77g, 0,25 mol) in Methanol (80 ml) und Wasser (4,5 ml) wurde auf 0 °C gekühlt und durch Zugabe von 30%-iger Natronlauge auf pH 9,6 gestellt. Dieser Suspension wurde innerhalb von 5 Minuten Pentanimidsäuremethylester (68,81g einer 42%-igen Lösung in Toluol = 0,25 mol) zugegeben. Nach 18 stündigem Rühren bei Raumtemperatur wurde die Suspension filtriert, der Filterkuchen mit Toluol (75ml) gewaschen und getrocknet.
Ausbeute: 25,45g (Gehalt > 95% nach H-NMR) 64 % bez. auf Glycin.
- ¹H-NMR (CH₃OD, 400 Mhz) δ: 0,94 (3H, t);
1,43 (2H, m);
1,70 (2H, m);
2,50 (2H, t);
3,75 (2H, s).

### Beispiel 2

### Herstellung von (Pentanimidoylamino)essigsäure

Eine gerührte Suspension von Glycin (18,77g, 0,25 mol) in Methanol (80 ml) und Wasser (4,5 ml) wurde auf 0 °C gekühlt und durch Zugabe von 30%-iger Natronlauge auf pH 9,6 gestellt. Dieser Suspension wurde innerhalb von 7 Minuten Pentanimidsäuremethylester (67,12 g einer 42,9%-igen Lösung in Toluol = 0,25 mol) zugegeben. Das Reaktionsgemisch wurde während 5 Stunden bei Raumtemperatur gerührt. Methanol und Wasser wurden danach bei einem reduziertem Druck von 30 mbar bis 150 mbar abdestilliert. Insgesamt wurden während dieser Destillation 250 ml Toluol zugegeben. Danach wurde filtriert, der Filterkuchen mit Toluol (75ml) gewaschen und getrocknet.
Ausbeute: 39,61g (Gehalt ca.90% nach H-NMR) 90 % bez. auf Glycin.

### Beispiel 3

### Herstellung von 2-Butyl-5-chlorimidazol-4-carbaldehyd

Zu einer Suspension von (Pentanimidoylamino)essigsäure (15,82g, 100mmol) in Toluol (75ml) wurde innerhalb von 5 Minuten Phosphoroxychlorid (43,80g, 280mmol) zudosiert. Das Gemisch wurde auf 80 °C erhitzt und darauf innert 7 Minuten mit N,N-Dimethylformamid (20,57g, 280mmol) versetzt. Die Temperatur stieg dabei auf 96 °C. Nach 2 Stunden Rühren bei 100 °C wurde auf 30 °C gekühlt. Das Reaktionsgemisch wurde darauf unter Rühren in 80 ml Wasser so eingetragen, dass die Temperatur stets unter 30 °C gehalten werden konnte. Nach Zugabe von Essigsäureethylester (80ml) und Celite (5g) wurde der pH des Gemisches mit 30%-iger Natronlauge auf 1,2 gestellt. Das Gemisch wurde filtriert, darauf erfolgte eine Phasentrennung bei 30 °C. Die organische Phase wurde zweimal mit Wasser gewaschen und darauf zur Trockene eingeengt. Das Titelprodukt wurde in einer Ausbeute von 13,39g (HPLC-Gehalt 81,4%) 58% bez. auf (Pentanimidoylamino)essigsäure erhalten.

### Beispiel 4

### Herstellung von 2-Butyl-5-chlorimidazol-4-carbaldehyd (ohne Isolation von (Pentanimidoylamino)essigsäure)

Eine weisse Suspension von 37,91 g (0,50 mol) Glycin, 9,0 g (0,50 mol) Wasser und 160 ml Methanol wurde auf 0 °C gekühlt. Der pH wurde durch Zugabe von 30%iger Natronlauge auf 9,5 gestellt. Innerhalb von 11 Minuten wurden 140,46 g einer Lösung von Pentanimidsäuremethylester (41%ig ≡ 0,50 mol) in Toluol zugetropft, so dass die Temperatur bei 0 °C gehalten werden konnte. Das Reaktionsgemisch wurde 20 h bei Raumtemperatur gerührt, wonach der pH durch Zugabe von konz. Schwefelsäure von 10,13 auf 7,0 gestellt wurde. 500 ml Toluol wurden zugegeben und Methanol und Wasser wurden unter Vakuum abdestilliert. Nach der Destillation wurden 72,6 g Toluol zugegeben. Die schwach gelbliche Suspension wurde auf 0 °C gekühlt und innerhalb von 11 Minuten mit 219,0 g (1,40 mol) Phosphoroxychlorid versetzt. Nach 20 Minuten wurde das Gemisch auf 80 °C erhitzt, mit 102,9 g (1,40 mol) Dimethylformamid versetzt, 2 h bei 100 °C erhitzt, auf 35 °C abkühlen gelassen und unter Rühren auf 350 ml Wasser gegossen, so dass die Temperatur unter 30 °C gehalten werden konnte. Das Gemisch wurde mit 300 ml Essigester und 20 g Celit versetzt und während 15 Minuten bei 25 - 30 °C gerührt. Der pH wurde mittels Zugabe von 275,0 ml 30%iger Natronlauge auf 1,20 gestellt. Das Celit wurde bei ca. 30 °C abgenutscht und die Phasen wurden bei ca. 30 °C getrennt. Die organische Phase wurde zweimal mit Wasser gewaschen und zur Trockene eingeengt.
Ausbeute: 68,1 g (HPLC-Gehalt 85,0%), 62% bez. auf Glycin.

### Beispiel 5

### Herstellung von (Benzimidoylamino)essigsäure

Eine weisse Suspension von Glycin (3,81 g, 50 mmol) in Wasser (0,9 g) und Methanol (16 ml) wurde bei Raumtemperatur gerührt, mittels Zugabe von Natronlauge auf pH 9,6 gestellt und mit Benzimidsäureethylester (7,69 g, 50 mmol) versetzt. Das Gemisch wurde 1 h bei 50 °C erhitzt und auf Raumtemperatur abkühlen gelassen. Der Feststoff wurde abfiltriert, mit Toluol gewaschen und bei Raumtemperatur / 30 mbar getrocknet.
Ausbeute: 6,81 g (ca. 95%ig nach H-NMR), 74% bez. auf Glycin.
- ¹H-NMR (D₂O, 400 MHz) δ: 4,09 (2H, s);
7,63 (2H, m);
7,76 (3H, m).

### Beispiel 6

### Herstellung von 5-Chlor-2-phenyl-3H-imidazol-4-carbaldehyd

Eine Suspension von (Benzimidoylamino)essigsäure (4,38 g, 25 mmol) in Toluol (25 ml) bei 0 °C wurde innerhalb von 5 Minuten mit Phosphoroxychlorid (10,73 g, 70 mmol) versetzt. Nach Zugabe von Toluol (19 ml) wurde das Gemisch auf 80 °C erhitzt, mit N,N-Dimethylformamid (5,12 g, 70 mmol) versetzt und 2 h bei 100 °C nachreagiert. Das Reaktionsgemisch wurde zu Wasser (19 ml) gegeben, sodass die Temperatur unter 30 °C gehalten werden konnte, und der Reaktionskolben wurde mit Essigester (15 ml) nachgespült. Das Reaktionsgemisch wurde mit Celit (2,25 g) versetzt, 0,5 h bei 25 °C gerührt, und mittels Zugabe von 30%iger Natronlauge (7,3 ml) auf pH 1,2 gestellt. Das Gemisch wurde filtriert, und die Phasen wurden getrennt. Die organische Phase wurde zweimal mit Wasser gewaschen und zur Trockene eingeengt.
Ausbeute: 4,13 g (ca. 95%ig nach H-NMR), 76% bez. auf (Benzimidoylamino)essigsäure.
- ¹H-NMR (CDCl₃, 400 MHz) δ: 7,5 (3H, m);
8,1 (2H, m);
9,77 (1H, s);
11,7 (1H, br. s).

## Patentansprüche

1. Verfahren zur Herstellung von 5-Chlorimidazol-4-carbaldehyden der allgemeinen Formel 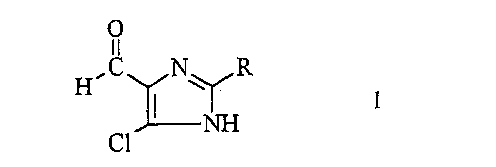 worin R Wasserstoff, eine geradkettige oder verzweigte (C₁-C₆)-Alkylgruppe, eine geradkettige oder verzweigte Alkenylgruppe mit bis zu sechs C-Atomen, eine Cyclopropyl-, Cylobutyl-, Cyclopentyl oder Cyclohexylgruppe oder eine gegebenenfalls mit (C₁-C₆)-Alkyl, Halogen, Nitro oder Amino substituierte Phenyl- oder Phenyl-(C₁-C₆)-alkylgruppe bedeutet, **dadurch gekennzeichnet, dass** in der ersten Stufe Glycin mit einem Imidsäureester der allgemeinen Formel 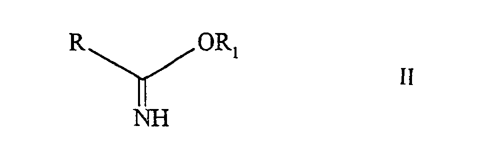 worin R die genannte Bedeutung hat und R₁ eine (C₁-C₆)-Alkylgruppe bedeutet, zu einer Verbindung der allgemeinen Formel 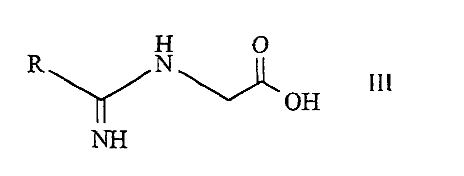 worin R die genannte Bedeutung hat, umgesetzt wird, und in der zweiten Stufe diese Verbindung mit einem Vilsmeier-Reagenz, welches zusammengesetzt ist aus einem Chlorierungsmittel und einem Formamid der allgemeinen Formel 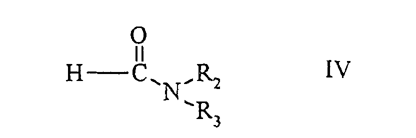 worin R₂ und R₃ gleich oder verschieden sind und eine (C₁-C₄)-Alkylgruppe oder eine Phenylgruppe bedeuten, zum Endprodukt überführt wird

2. Verfahren nach Anspruch 1, **dadurch gekennzeichnet, dass** die Verbindung der allgemeinen Formel III nicht isoliert wird.

3. Verfahren nach Anspruch 1 oder 2, **dadurch gekennzeichnet, dass** die Umsetzung in der ersten Stufe bei einem pH-Wert zwischen 4 bis 12 und bei einer Temperatur zwischen -20 °C und 80 °C durchgeführt wird.

4. Verfahren nach einem der Ansprüche 1 bis 3, **dadurch gekennzeichnet, dass** als Chlorierungsmittel im Vilsmeier-Reagenz Phosphoroxychlorid eingesetzt wird.

5. Verfahren nach einem der Ansprüche 1 bis 4, **dadurch gekennzeichnet, dass** als Formamid der allgemeinen Formel IV im Vilsmeier-Reagenz N,N-Dimethylformamid verwendet wird.

6. Verfahren nach einem der Ansprüche 1 bis 5, **dadurch gekennzeichnet, dass** das Molverhältnis Chlorierungsmittel zu Formamid der allgemeinen Formel IV im Vilsmeier-Reagenz zwischen 1 zu 1 und 4 zu 1 liegt.

7. Verfahren nach einem der Ansprüche 1 bis 5, **dadurch gekennzeichnet, dass** die Umsetzung mit dem Vilsmeier-Reagenz bei einer Temperatur zwischen 60 °C und 200 °C erfolgt.

## Claims

1. Process for the preparation of 5-chloroimidazole-4-carbaldehydes of the general formula 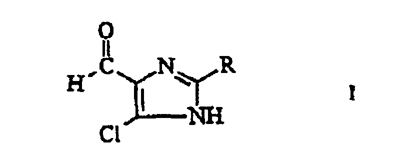 wherein R represents hydrogen, a straight-chain or branched (C₁-C₆)-alkyl group, a straight-chain or branched alkenyl group having up to six carbon atoms, a cyclopropyl, cyclobutyl, cyclopentyl or cyclohexyl group, or a phenyl or phenyl-(C₁-C₆)-alkyl group optionally substituted by (C₁-C₆)-alkyl, halogen, nitro or by amino, which process is **characterised in that** in the first step glycine is reacted with an imido acid ester of the general formula 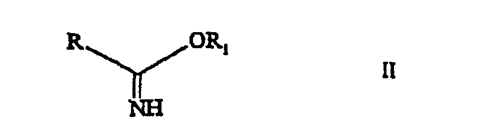 wherein R is as defined above and R₁ represents a (C₁-C₆)-alkyl group, to form a compound of the general formula 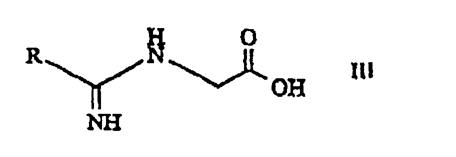 wherein R is as defined above, and in the second step that compound is converted into the end product with a Vilsmeier reagent, which is composed of a chlorinating agent and a formamide of the general formula 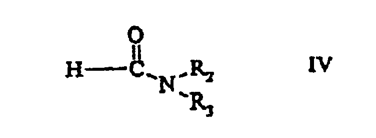 wherein R₂ and R₃ are identical or different and represent a (C₁-C₄)-alkyl group or a phenyl group.

2. Process according to claim 1, **characterised in that** the compound of the general formula III is not isolated.

3. Process according to claim 1 or 2, **characterised in that** the reaction in the first step is carried out at a pH value of from 4 to 12 and at a temperature of from -20°C to 80°C.

4. Process according to any one of claims 1 to 3, **characterised in that** phosphorus oxychloride is used as the chlorinating agent in the Vilsmeier reagent.

5. Process according to any one of claims 1 to 4, **characterised in that** N,N-dimethylformamide is used as the formamide of the general formula IV in the Vilsmeier reagent.

6. Process according to any one of claims 1 to 5, **characterised in that** the molar ratio of chlorinating agent to formamide of the general formula IV in the Vilsmeier reagent is from 1:1 to 4:1.

7. Process according to any one of claims 1 to 5, **characterised in that** the reaction with the Vilsmeier reagent is carried out at a temperature of from 60°C to 200°C.

## Revendications

1. Procédé de préparation de 5-chloroimidazole-4-carbaldéhydes de formule générale 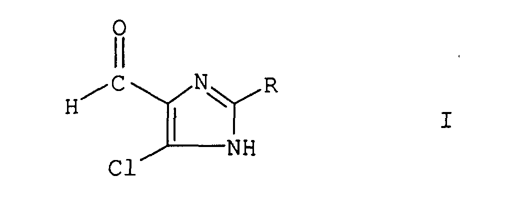 où R représente l'hydrogène, un groupe alkyle en C₁-C₆ linéaire ou ramifié, un groupe alcényle linéaire ou ramifié ayant jusqu'à six atomes de carbone, un groupe cyclopropyle, cyclobutyle, cyclopentyle ou cyclohexyle ou un groupe phényle ou phényl-alkyle en C₁-C₆ éventuellement substitué par alkyle en C₁-C₆, halogène, nitro ou amino, **caractérisé en ce que**, dans la première étape, la glycine est convertie avec un ester d'acide imidique de formule générale 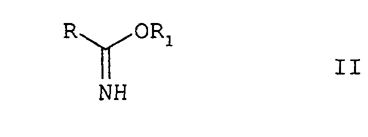 où R a la signification citée et R₁ représente un groupe alkyle en C₁-C₆, en un composé de formule générale 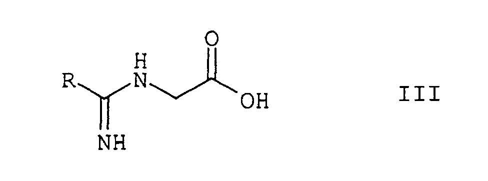 où R a la signification citée, et dans la seconde étape ce composé est converti en le produit final avec un réactif de Vilsmeier qui est constitué par un agent de chloration et un formamide de formule générale 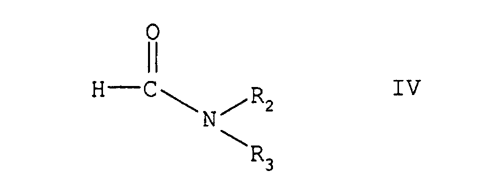 où R₂ et R₃ sont identiques ou différents et représentent un groupe alkyle en C₁-C₄ ou un groupe phényle.

2. Procédé selon la revendication 1 **caractérisé en ce que** le composé de formule générale III n'est pas isolé.

3. Procédé selon la revendication 1 ou 2 **caractérisé en ce que** la réaction est conduite dans la première étape à un pH de 4 à 12 et à une température de -20°C à 80°C.

4. Procédé selon l'une des revendications 1 à 3 **caractérisé en ce que** l'oxychlorure de phosphore est utilisé comme agent de chloration dans le réactif de Vilsmeier.

5. Procédé selon l'une des revendications 1 à 4 **caractérisé en ce que** le N,N-diméthylformamide est utilisé comme formamide de formule générale IV dans le réactif de Vilsmeier.

6. Procédé selon l'une des revendications 1 à 5 **caractérisé en ce que** le rapport molaire de l'agent de chloration au formamide de formule générale IV dans le réactif de Vilsmeier est de 1:1 à 4:1.

7. Procédé selon l'une des revendications 1 à 5 **caractérisé en ce que** la réaction avec le réactif de Vilsmeier a lieu à une température de 60°C à 200°C.
